Europäisches Patentamt

European Patent Office

Office européen des brevets

(11) Veröffentlichungsnummer: **0 393 543**
**A2**

(12) **EUROPÄISCHE PATENTANMELDUNG**

(21) Anmeldenummer: **90107134.0**

(22) Anmeldetag: **13.04.90**

(51) Int. Cl.⁵: **A61F 2/30, A61F 2/32**

(30) Priorität: **19.04.89 CH 1488/89**

(43) Veröffentlichungstag der Anmeldung:
**24.10.90 Patentblatt 90/43**

(84) Benannte Vertragsstaaten:
**DE FR GB IT**

(71) Anmelder: **Emil Schenker AG**
**Stauwehrstrasse 34**
**CH-5012 Schönenwerd(CH)**

(72) Erfinder: **Eichler, Joachim, Prof. Dr.**
**Gabelsbornstrasse 28**
**D-6200 Wiesbaden(DE)**
Erfinder: **Bischof, Richard**
**Allmendstrasse 34**
**CH-4658 Däniken(CH)**

(74) Vertreter: **Fillinger, Peter, Dr.**
**Rütistrasse 1a**
**CH-5400 Baden(CH)**

(54) **Gelenkpfanne zu einer Hüftgelenkprothese.**

(57) Die Gelenkpfanne (1) weist eine gerundete Aussenfläche (13) und mindestens zwei parallele, zentralsymmetrisch über die Aussenfläche vorstehende Bolzen (6) zur Verdrehsicherung auf. Damit die Gelenkpfanne (1) nach dem Einbau sofort belastbar ist, wird vorgeschlagen, dass die Bolzen (6) eine durch die Gelenkpfannenwand gehende, längsachsiale Bohrung (7) aufweisen, welche gegen die Gelenkpfannenvorderseite (8) offen ist. Die Bohrung (7) weist im Bereich (11) der Bolzen (6) einen kleineren Querschnitt auf als im Bereich (9, 10) der Gelenkpfannenwand, so dass beim Einschieben eines Stiftes (13) in die Bohrung (7) von der Gelenkpfannenvorderseite her die Bolzen (6) in radialer Richtung nach Art eines Spreizdübels gespreizt werden.

Fig. 1

## Gelenkpfanne zu einer Hüftgelenkprothese

Die vorliegende Erfindung bezieht sich auf eine Gelenkpfanne gemäss dem Oberbegriff des Anspruchs 1.

Derartige Gelenkpfannen werden beim Einpflanzen in entsprechende Kavitäten des Beckenknochens gesteckt, welche, wenn nach dem Einbau eine zureichende Belastbarkeit und Unverrückbarkeit gegeben sein soll, sehr genau gefertigt sein müssen.

Die vorliegende Erfindung stellt sich die Aufgabe, eine Gelenkpfanne der erwähnten Art derart zu verbessern, dass die Ausnehmungen für die Aufnahme der Bolzen mit Toleranzen gefertigt sein können, und dass die Belastbarkeit nach dem Einbau trotzdem grösser als bei den bekannten Gelenkpfannen ist.

Erfindungsgemäss wird diese Aufgabe gelöst durch die kennzeichnenden Merkmale des Anspruchs 1.

Anhand der beiliegenden schematischen Zeichnung wird die Erfindung beispielsweise erläutert. Es zeigen:

Fig. 1 einen Querschnitt durch ein erstes Ausführungsbeispiel einer Gelenkpfanne,

Fig. 2 eine Frontansicht der Gelenkpfanne nach Fig. 1,

Fig. 3 einen Querschnitt durch ein zweites Ausführungsbeispiel einer Gelenkpfanne und

Fig. 4 eine Frontansicht zum Ausführungsbeispiel nach Fig. 3.

Die Zeichnung zeigt eine Gelenkpfanne 1 in die die Gelenkkugel des nicht gezeigten Hüftprothesenschaftes eingelegt wird. Die vorzugsweise aus Kunststoff (Polyäthylen) bestehende Gelenkpfanne 1 weist im wesentlichen die Form eines Rotationskörpers mit kalottenförmiger Gelenkfläche 2 auf. Die ebenfalls gerundete Aussenfläche 3 ist mit einer zur Rotationsachse konzentrischen Rillung 4 versehen, die das Verwachsen der eingepflanzten Gelenkpfanne 1 mit dem Beckenknochen begünstigen soll. Um ein Verdrehen der eingepflanzten Gelenkpfanne 1 im Beckenknochen zu vermeiden, überragen drei um die Rotationsachse 5 zentralsymmetrisch verteilte, parallele Bolzen 6 die Pfannenaussenfläche 3. Jeder Bolzen 6 ist längsachsial mit einer Bohrung 7 durchsetzt, die in die ringförmige Frontfläche 8 der Gelenkpfanne 1 mündet. Die Bohrung 7 weist in einem ersten, an die Frontfläche 8 anschliessenden Abschnitt 9 einen grösseren Durchmesser als im angrenzenden Mittelabschnitt 10 auf. Dessen Durchmesser wiederum ist grösser als im anschliessenden, bis zum Bolzenende führenden Endabschnitt 11, wo die Bolzenwand mit mindestens einem Schlitz 12 durchbrochen ist.

Für das Einsetzen der Gelenkpfanne 1 wird mit einem Bohrwerkzeug im Beckenknochen die aufnehmende Kavität herausgearbeitet. Anschliessend werden mittels einer Lehre drei Bohrungen für die Aufnahme der Bolzen 6 angebracht, worauf die Gelenkpfanne 1 eingesetzt werden kann. Dabei kommen die Bolzen 6 möglichst spielfrei in die erwähnten Bohrungen zu liegen und verhindern ein Verdrehen der Gelenkpfanne relativ zum Beckenknochen. Um die Gelenkpfanne 1 mechanisch in ihrer Lage zu sichern, werden in jede Bohrung 7 je ein Stift 13 geschlagen, bis der Stiftkopf 14 versenkt im ersten Bohrungsabschnitt 9 liegt. Der Durchmesser des Stiftes 13 entspricht im wesentlichen dem Bohrungsdurchmesser im Mittelabschnitt 10 und ist etwas kleiner als dieser aber grösser als im dritten Abschnitt. Damit kann er mühelos in die Bohrung 7 eingeschoben werden bis sein vorderes Ende am Endabschnitt 11 ansteht. Die Länge des Stiftes 13 ist vorzugsweise gleich oder unwesentlich kleiner als die Bohrungslänge. Beim Eintreiben des Stiftes in die Bohrung 7 trifft er somit auf den im Durchmesser kleineren Endabschnitt 11 und spreizt den Bolzen 6 radial auf, dessen Nachgiebigkeit durch die erwähnte schlitzförmige Durchbrechung 12 gewährleistet ist. Nach dem Eintreiben aller Stifte 13 ist die Gelenkpfanne 1 belastbar.

Um die Gewebeverträglichkeit der Gelenkpfanne zu verbessern, ist sie aussenseitig (einschliesslich der Bolzen) mit einer Metallschicht überzogen, welche galvanisch aufgebracht, aufgedampft oder aufgespritzt sein kann.

Um bei geschwächten Beckenknochen dessen Belastbarkeit durch die Gelenkpfanne 1 auf Druck zu verbessern, kann sie zweiteilig aus ineinander liegenden Schalen 15, 16 gebildet sein. Die innere Schale 15 ist wie die beschriebene Gelenkpfanne 1 ausgebildet, wobei indessen die Wandstärke etwas kleiner gewählt sein kann. Sie liegt mit ihrer Aussenfläche 17 spielfrei an der Innenfläche 18 der äusseren Metallschale 16, deren gerundete Aussenfläche 19 in die am Hüftknochen auszunehmende Kavität zu liegen kommt. Die drei Bolzen 6 sind wie beim ersten Ausführungsbeispiel gestaltet und einstückig mit der Kunststoffinnenschale 15 gefertigt und achsial durch entsprechende Bohrungen 20 in der äusseren Schale 16 hindurchgeführt.

Der Einbau erfolgt in der beschriebenen Weise. Die höhere Belastbarkeit der Gelenkpfanne 1 wird dadurch erreicht, dass der Rand der äusseren Schale 16 mit einem Flansch 21 versehen ist, der sich bei eingesetzter Gelenkpfanne 1 am Rand der Kavität gegen den Beckenknochen legt.

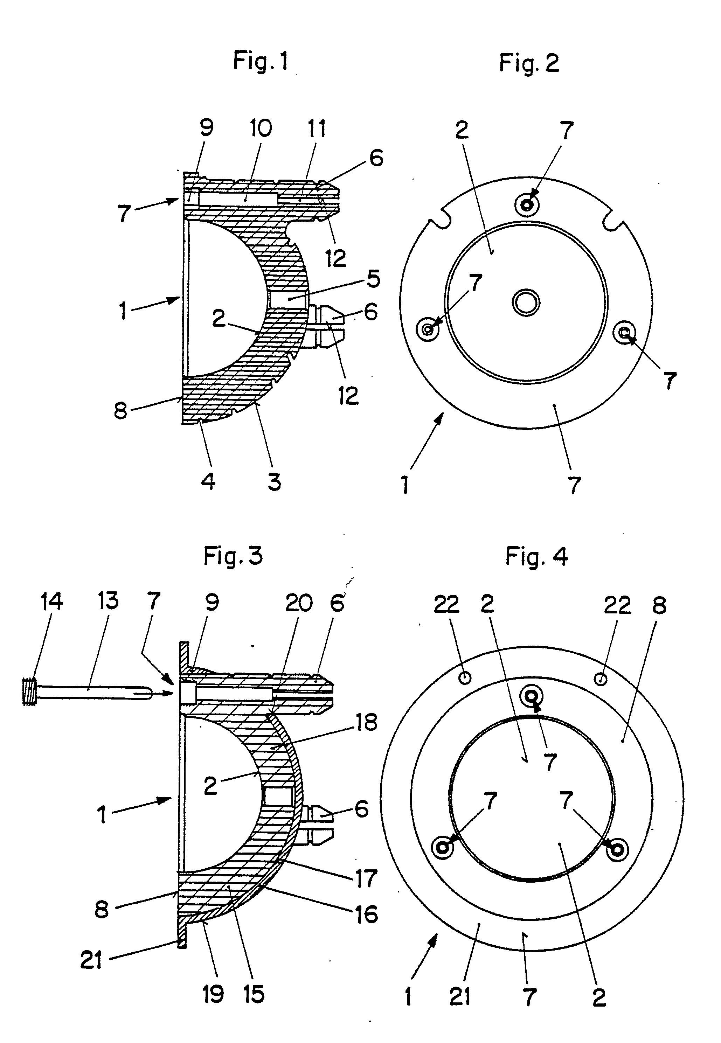

Der Flansch 21 kann, wie Fig. 4 zeigt mit weiteren, zentralsymmetrisch verteilten Bohrungen 22 versehen sein, durch welche Knochenschrauben in den Beckenknochen schraubbar oder in denen Werkzeuge ansetzbar sind.

Der Stiftkopf 14 ist vorzugsweise als Inbusschraube gestaltet und mit einem Selbstschneidegewinde versehen, welches in den ersten Abschnitt 9 der Bohrung 7 geschraubt wird. Dadurch ist ein ungewolltes Lösen des Stiftes 13 ausgeschlossen und es ergibt sich eine einfache Möglichkeit, die versenkten Stifte 13 zu lösen und herauszuziehen, wenn die Gelenkpfanne 1 auszuwechseln ist.

**Ansprüche**

1. Gelenkpfanne (1) zu einer Hüftgelenkprothese mit einer gerundeten, eine Rotationsfläche bildenden Aussenfläche (13, 19) und mindestens zwei parallelen, zentralsymmetrisch über die Aussenfläche vorstehenden Bolzen (6) zur Verdrehsicherung im eingepflanzten Zustand, dadurch gekennzeichnet, dass die Bolzen (6) eine durch die Gelenkpfannenwand gehende, längsachsiale Bohrung (7) aufweisen, welche gegen die Gelenkpfannenvorderseite (8) offen ist, dass die Bohrung (7) im Bereich (11) der Bolzen (6) einen kleineren Querschnitt aufweist als im Bereich (9, 10) der Gelenkpfannenwand und dass beim Einschieben eines Stiftes (13) in die Bohrung (7) von der Gelenkpfannenvorderseite her die Bolzen (6) in radialer Richtung nach Art eines Spreizdübels spreizbar sind.

2. Gelenkpfanne nach Anspruch 1, dadurch gekennzeichnet, dass die Bolzenwand mit einem Längsschlitz (12) versehen ist.

3. Gelenkpfanne nach Anspruch 1, dadurch gekennzeichnet, dass ihre Aussenfläche (3) und/oder die Bolzenaussenflächen gerillt (4) oder geriffelt sind.

4. Gelenkpfanne nach Anspruch 1, dadurch gekennzeichnet, dass sie aus Kunststoff besteht und einschliesslich der Bolzen (6) aussenseitig bevorzugt mit einer Metallschicht versehen ist.

5. Gelenkpfanne nach einem der Ansprüche 1 bis 3, dadurch gekennzeichnet, dass sie eine Aussenschale (16) aus Metall und einen die Gelenkfläche (2) bildenden Kunststoffkern (15) aufweist, mit dem die Bolzen (6) einstückig gefertigt sind, und dass die Aussenschale (16) mit den Bolzen (6) entsprechenden Durchbrechungen (20) versehen ist, derart, dass der Kunststoffkern (15) und die Aussenschale (16) mit Passsitz ineinander steckbar sind.

6. Gelenkpfanne nach Anspruch 5, dadurch gekennzeichnet, dass die Aussenschale (16) an ihrem Rand mit einem Stützflansch (21) versehen ist.

EP 0 393 543 A2

Fig. 1

Fig. 2

Fig. 3

Fig. 4